# EUROPÄISCHE PATENTSCHRIFT

(11) **EP 0 746 628 B1**
(45) Veröffentlichungstag und Bekanntmachung des Hinweises auf die Patenterteilung: **18.11.1998**
(21) Anmeldenummer: 95908271.0
(22) Anmeldetag: 13.02.1995
(51) Int. Cl.: C14C 9/02

(54) **VERWENDUNG SULFIERTER SUBSTANZEN ZUR FETTUNG VON LEDER**
USE OF SULPHITED SUBSTANCES FOR STUFFING LEATHER
UTILISATION DE SUBSTANCES SULFITEES POUR NOURRIR LE CUIR

(30) Priorität: 21.02.1994 DE 4405417
(43) Veröffentlichungstag der Anmeldung: 11.12.1996
(73) Patentinhaber: Henkel Kommanditgesellschaft auf Aktien, 40191 Düsseldorf (DE)
(72) Erfinder: ZAUNS-HUBER, Rudolf, D-40589 Düsseldorf (DE); MAINX, Hans-Georg, D-42700 Leichlingen (DE); SCHENKER, Gilbert, D-40699 Erkrath (DE)
(86) Internationale Anmeldenummer: EP9500509
(87) Internationale Veröffentlichungsnummer: WO9522630

(56) Entgegenhaltungen:
- EP-A- 0 178 557
- EP-A- 0 353 704

## Beschreibung

### Gebiet der Erfindung

Die Erfindung betrifft die Verwendung spezieller sulfierter Substanzen zur Fettung von Leder. Bei diesen Substanzen handelt es sich um Umsetzungsprodukte von Estern a) einwertiger Alkohole mit 1 bis 24 C-Atomen und Dicarbonsäuren mit 2 bis 24 C-Atomen, wobei die Summe der C-Atome der Alkohol- und Fettsäurebausteine der Ester mindestens 16 beträgt, mit einem Sulfierreagens aus mindestens einer organischen Sulfonsäure und Schwefelsäure.

### Stand der Technik

Neben den Gerbstoffen sind Fettungsmittel die wichtigsten Hilfsmittel, um den Charakter von Leder zu prägen. Die Wirkung der Fettungsmittel kommt durch eine faserisolierende Schmierung und durch eine Hydrophobierung zustande. Durch Umhüllung der Lederfasern mit einem Fettfilm wird die gegenseitige Reibung verringert und demzufolge die Geschmeidigkeit und Dehnbarkeit des Gewebes verbessert. Das hat positive Auswirkungen auf die Reißfestigkeit des Leders, denn in einem dehnbaren Werkstoff richten sich viele Fasern bei Zugbeanspruchung in der Zugrichtung aus und setzen dann dem Zerreißen einen größeren Widerstand entgegen als dieselben Fasern innerhalb eines spröden Werkstoffes.

Als Lederfettungsmittel werden im allgemeinen pflanzliche und tierische Öle, Fette und Wachse eingesetzt, ferner die aus diesen Stoffen durch chemische Umwandlung gewonnenen Hydrolyse-, Sulfierungs-, Oxidations- und Härtungsprodukte und schließlich mineralische Fettungsmittel; im einzelnen:

Die verseifbaren Fette und Öle sowie die natürlichen Wachse und Harze gehören zu den Estern. Unter Ölen und Fetten werden dabei vom Lederfachmann Ester aus Glycerin und Fettsäuren bezeichnet, die bei Raumtemperatur fest bzw. flüssig sind. Zur Lederfettung werden dabei aus der Gruppe der tierischen Fette insbesondere Trane, Fischöl, Rindertalg und Rinderklauenöl, aus der Gruppe der pflanzlichen Fette Rizinusöl, Rüböl und Leinöl herangezogen. In Wachsen und Harzen sind die Fettsäuren statt mit Glycerin mit höhermolekularen Alkoholen verestert. Beispiele für Wachse sind Bienenwachs, chinesisches Wachs, Carnaubawachs, Montanwachs und Wollfett; zu den wichtigsten Harzen zählen Kolophonium, Juchtenöl und Schellack.

Durch chemische Umwandlung pflanzlicher und tierischer Fette erhält man Produkte, die wasserlöslich sind und die darüber hinaus in unterschiedlichem Maße emulgierend auf wasserunlösliche Fettstoffe wirken. Bekannt sind etwa die sulfierten wasserlöslichen Öle verschiedenster Art, die durch Oxidation veränderten Trane, die als Degras oder Moellon bezeichnet werden, ferner die Seifen, die bei der hydrolytischen Spaltung natürlicher Fette entstehen, gehärtete Fette sowie schließlich freie Fettsäuren wie Stearinsäure als Einbrennfette. Die meisten tierischen und pflanzlichen Fette weisen eine gewisse Affinität zur Ledersubstanz auf, die durch die Einführung oder Freilegung hydrophiler Gruppen noch beträchtlich gesteigert wird.

Wichtig für die Lederherstellung sind weiter die mineralischen Fettungsmittel. Diese Kohlenwasserstoffe sind den natürlichen Fetten und Ölen in manchen Eigenschaften ähnlich, lassen sich jedoch nicht verseifen. Es handelt sich um Fraktionen der Erdöldestillation, die in flüssiger Form Mineralöl, in pastöser Form Vaseline und in fester Form Paraffin genannt werden.

Die Fettung von Leder erfolgt üblicherweise mit Hilfe von Öl-in-Wasser-Emulsionen, den sogenannten Lickerölen. Diese Likeröle sind selbstemulgierende Produkte, die in wäßrigem Medium ein Neutralöl sowie einen Emulgator enthalten. Dabei kann der Emulgator dem Lickeröl entweder als separate Komponente beigemischt sein oder durch partielle Sulfierung des Neutralöls hergestellt worden sein.

Die mit Abstand wichtigste Gruppe von Fettungsmitteln sind die anionischen. Sie umfaßt sulfatierte, sulfitierte, sulfonierte und sulfochlorierte Öle. Die klassische Sulfatierung erfolgt dabei mit konzentrierter Schwefelsäure. Die Reaktion erfolgt dabei - je nach der Art des eingesetzten Öls - zum einen an freien Hydroxylgruppen, die entweder primär vorhanden sein können wie im Falle des Ricinusöls, oder die sekundär gebildet werden durch partielle Hydrolyse des Triglycerids, zum anderen durch Addition an -C=C-Doppelbindungen ungesättigter Fettsäurereste (vergl. Hans Herfeld, Hrsg., "Bibliothek des Leders, Frankfurt 1987, Band 4, Seite 76-77).

Beispiele für Mittel zur fettenden Ausrüstung von Leder, die nach diesem klassischen und nach wie vor wichtigen Verfahrens hergestellt werden, sind sulfatiertes Klauenöl, sulfatiertes Lardöl und Rapsöl, sulfatiertes Fischöl und sulfatierter Rindertalg. Die dabei zur Sulfatierung typischerweise eingesetzten Mengen an konzentrierter Schwefelsäure liegen typischerweise im Bereich von 15 bis 60 Gew.-% - bezogen auf das zu sulfatierende Öl (vergl. Hans Herfeld, Hrsg., "Bibliothek des Leders, Frankfurt 1987, Band 4, Seite 77-78).

Das klassische Sulfatierungsverfahren hat jedoch den Nachteil, daß es aufwendige und zeitraubende Aufarbeitungsmaßnahmen erfordert. Der Grund liegt darin, daß die zu sulfatierenden Fette und Öle in der Praxis mit - bezogen auf diese Fette und Öle - den bereits erwähnten hohen Mengen an konzentrierter Schwefelsäure, bei Fischöl z.B. mit ca. 20 Gew.-%, in Kontakt gebracht werden. Dabei reagiert nur ein Teil der Schwefelsäure, der Rest - in der Regel der überwiegende Teil - liegt nach der Reaktion in unveränderter Form vor. Nach der Neutralisation mit üblicherweise NaOH liegt dementprechend anschließend eine große Menge an Natriumsulfat vor. Die Folge davon ist, daß die Produkte nicht lagerstabil sind. Unter mangelnder Lagerstabilität ist zu verstehen, daß eine Trennung in mehrere Phasen auftritt. Die wäßrige Phase enthält dabei einen großen Teil der jeweiligen anorganischen Salze, z. B. Natriumsulfat oder Amoniumsulfat.

Es ist daher üblich, die Produkte, die nach dem klassischen Sulfatierungsverfahren zugänglich sind, einer speziellen Nachbereitung zu unterziehen. Die Nachbereitung dient dabei dem Zweck, einen großen Teil des Salzes zu entfernen. Dies geschieht üblicherweise dadurch, daß man das neutralisierte Sulfatierungsprodukt zunächst mit Wasser wäscht. Anschließend trennt man die (salzreiche) wäßrige Phase von der (salzarmen) Ölphase. Der Vorgang der Phasentrennung ist jedoch in der Praxis schwierig durchzuführen und zeitraubend. Darüber hinaus sind die dabei anfallenden Waschwässer umweltbelastend sowohl im Hinblick auf die darin enthaltene Salzfracht, als auch im Hinblick an die darin enthaltenen (geringen) Mengen an sulfatierten Produkten. Somit entspricht die im Zuge der klassischen Sulfatierverfahren erforderliche Nachbereitung weder in ökonomischer noch in ökologischer Hinsicht den Anforderungen, die heutzutage an moderne und effiziente und dabei gleichzeitig möglichst umweltverträgliche Produktionsmethoden gestellt werden.

Aus der EP-A-247 509 ist bekannt, daß sich Sulfierungsprodukte oxalkylierter natürlicher Fette und Öle zur Fettung von Leder eignen. Als Sulfier-Reagenzien werden dabei Schwefelsäure und gasförmiges Schwefeltrioxid genannt. In bezug auf den Einsatz von Schwefelsäure als Sulfierreagens dürfte diese Methode im wesentlichen dem aus IN-A-146 476 bekannten Verfahren entsprechen, bei dem Froschöl nach Ethoxylierung sulfatiert wird, ein Verfahren, das darauf abzielt, die Gebrauchseigenschaften sulfatierter Öle, vor allem die Elektrolytbeständigkeit durch Modifikation des Rohstoffs vor der Sulfierung zu verbessern, das jedoch in der einschlägigen Fachliteratur eher als Kuriosität gewertet wurde (vergl. Hans Herfeld, Hrsg., "Bibliothek des Leders, Frankfurt 1987, Band 4, Seite 78, fünfter Absatz).

Aus der DE-A-41 41 532 ist ein Verfahren zur Herstellung hydrophylisierter Triglyceride bekannt, bei dem gesättigte, ungesättigte und/oder geblasene Triglyceride zunächst in Gegenwart von Glycerin und alkalischen Katalysatoren mit Ethylenoxid umsetzt, die resultierenden ethoxylierten Triglyceride mit gasförmigen Schwefeltrioxid sulfiert und die daraus resultierenden sauren Sulfierprodukte anschließend mit wäßrigen Basen neutralisiert. Nach der Lehre der DE-A-41 41 532 eignen sich die so hergestellten Produkte zur Fettung von Leder.

Ein für die Praxis sehr wichtiges Bedürfnis besteht darin, fettende Substanzen bzw. Ausrüstungsmittel zur Verfügung zu stellen, die in der gegerbten Hautsubstanz so zuverlässig gebunden werden können, daß eine für die praktischen Bedürfnisse hinreichende Wasch- und Reinigungsbeständigkeit der Leder- und Pelzwaren sichergestellt ist. Hochwertige Lederwaren, beispielsweise aus der Bekleidungsindustrie, sollen dabei sowohl der wäßrig-tensidischen Wäsche als auch gegebenenfalls einer chemischen Reinigung ohne wesentliche Qualitätseinbuße zugänglich sein.

Schließlich besteht für Leder, die im Innenbereich von Autos und Flugzeugen Verwendung finden, Bedarf, über Substanzen zur fettenden Ausrüstung zu verfügen, die Fogging-echt sind. Unter "Fogging" ist zu verstehen, daß im Laufe der Zeit flüchtige Substanzen aus dem Leder entweichen und sich in unerwünschter Weise niederschlagen, z.B. auf Windschutzscheiben. Unter Fogging-echten Substanzen ist zu verstehen, daß diese Substanzen zum einen selbst so fest im Innern des Leders gebunden sind, daß sie praktisch nicht flüchtig sind, zum anderen, daß diese Substanzen die Fogging-Charakteristik üblicher Fettungsmittel bzw. Fettungsmittelbestandteile verbessern, d.h. deren Fogging-Werte reduzieren.

Ein Verfahren zur Herstellung von Leder mit verbesserter Fogging-Charakteristik ist etwa in EP-A-498 634 beschrieben. Dabei lehrt die EP-A-498 634 eine Behandlung des Leders mit wäßrigen Dispersionen, die frei sind von organischen Lösungsmitteln und die ein amphiphiles Copolymer enthalten, das aus wenigstens einem hydrophilen Monomer und wenigstens einem hydrophoben Monomer besteht.

### Beschreibung der Erfindung

Aufgabe der vorliegenden Erfindung war es, Substanzen bereitzustellen, die sich zur fettenden Ausrüstung von Leder eignen. Unter dem Begriff der "fettenden Ausrüstung" ist dabei einerseits die Lederfettung im engeren Wortsinne zu verstehen, als auch die Hydrophobierung von Leder.

Eine weitere Aufgabenstellung war es, daß diese Substanzen dem damit behandelten, das heißt gelickerten Leder gute Eigenschaften hinsichtlich Narbenfestigkeit, Fülle, Schmalzigkeit verleihen und daß anschließend gefärbte Leder sich durch einen nicht aufgehellten, egalen Farbton auszeichnen.

Eine weitere Aufgabenstellung war es, daß diese Substanzen gut von Leder aufgenommen werden und sich insbesondere durch eine hohe Lickerflottenauszehrung auszeichnen. Der letztgenannte Punkt ist neben der rein technischen Relevanz auch unter ökologischen Gesichtspunkten von Nutzen.

Eine weitere Aufgabenstellung war es, daß diese Substanzen sich durch eine gute Lagerstabilität auszeichnen.

Eine weitere Aufgabenstellung war es, daß sich die mit diesen Substanzen behandelten Leder durch eine gute Fogging-Charakteristik auszeichnen.

Eine weitere Aufgabenstellung war es, die angestrebten Substanzen in einem einfachen, leicht durchführbaren Verfahren zugänglich zu machen. Dieser Punkt ist insbesondere im Hinblick auf die oben genannte große Gruppe von anionischen Lederfettungsmitteln von Bedeutung, die durch Umsetzung von Fetten und Ölen mit konzentrierter Schwefelsäure hergestellt werden und für die in der Praxis eine Nachbereitung der Rohprodukte (Waschvorgänge in Kombination mit Phasentrennung) üblich ist.

Überraschenderweise wurde nun gefunden, daß spezielle sulfierte Substanzen, die erhältlich sind durch Umsetzung von Estern a) einwertigen Alkoholen mit 1 bis 24 C-Atomen und Dicarbonsäuren mit 2 bis 24 C-Atomen, wobei die Summe der C-Atome der Alkohol- und Fettsäurebausteine der Ester mindestens 16 beträgt, mit einem Sulfierreagens b) aus mindestens einer organischen Sulfonsäure b1) und Schwefelsäure b2), mit der Maßgabe, daß der Anteil der Schwefelsäure - bezogen auf die Summe von organischer Sulfonsäure und Schwefelsäure - 0,5 bis 80 Gew.-% beträgt, nebst anschließender Neutralisation, die genannten Anforderungen in jeder Hinsicht ausgezeichnet erfüllen.

Von besonderem Vorteil ist es, daß im Zuge der Herstellung der erfindungsgemäß einzusetzenden sulfierten Substanzen keine aufwendigen Nachbehandlungen erforderlich sind. Ausdrücklich sei in diesem Zusammenhang darauf hingewiesen, daß das Verfahren gemäß der erfindungsgemäßen Lehre dem klassischen Sulfatierungsverfahren sowohl im Hinblick auf die Verfahrensökonomie, als auch im Hinblick auf ökologische Aspekte deutlich überlegen ist. So sind zur Herstellung von Sulfierungsprodukten nach den erfindungsgemäßen Verfahren typischerweise 2 bis 4 Stunden erforderlich, während das klassische Sulfatierungsverfahren - das wie beschrieben eine aufwendige Nachbehandlung erforderlich macht - üblicherweise 8 bis 24 Stunden erfordert. In ökologischer Hinsicht ist das Verfahren gemäß der vorliegenden Erfindung dem klassischen Sulfatierungsverfahren deshalb überlegen, weil keine bzw. deutlich geringere Mengen an umweltbelastenden Abwässern bzw. Abluftgasen anfallen.

Gegenstand der vorliegenden Erfindung ist dementsprechend die Verwendung sulfierter Substanzen zur fettenden Ausrüstung von Leder, wobei die sulfierten Substanzen erhältlich sind durch Umsetzung von Estern a) von einwertigen Alkoholen mit 1 bis 24 C-Atomen und Dicarbonsäuren mit 2 bis 24 C-Atomen, wobei die Summe der C-Atome der Alkohol- und Fettsäurebausteine der Ester mindestens 16 beträgt, mit einem Sulfierreagens b) nebst anschließender Neutralisation. Als Sulfierreagens b) setzt man dabei eine Kombination aus mindestens einer organischen Sulfonsäure b1) und Schwefelsäure b2) ein, mit der Maßgabe, daß der Anteil der Schwefelsäure - bezogen auf die Summe von organischer Sulfonsäure und Schwefelsäure - 0,5 bis 80 Gew.-% beträgt.

Im Rahmen der vorliegenden Erfindung sind unter "Dicarbonsäuren" alle zweibasischen organischen Carbonsäuren mit insgesamt 2 bis 24 C-Atomen zu verstehen. Bevorzugt sind dabei diejenigen Dicarbonsäuren, die sich - im Sinne der klassischen Definition etwa des "Römpp" (vergl. z.B. O.-A.Neumüller, Römpps Chemie-Lexikon, Stuttgart 1973, S.828f) - von linearen Paraffinen dadurch ableiten, daß deren beide Enden zu Carboxylgruppen oxidiert sind. Beispiele für geeignete Dicarbonsäuren sind Oxalsäure, Malonsäure, Bernsteinsäure, Glutarsäure, Adipinsäure, Pimelinsäure, Korksäure, Azelainsäure, Sebacinsäure, Dodecandisäure, ferner Maleinsäure, Fumarsäure, Phthalsäure und Terephthalsäure. Es ist jedoch auch möglich, substituierte Dicarbonsäuren einzusetzen. Dabei sind OH-Gruppen enthaltende Dicarbonsäuren, z.B. Äpfelsäure, bevorzugt.

In einer weiteren Ausführungsform der vorliegenden Erfindung gilt die zusätzliche Maßgabe, daß die Komponente a) in Bezug auf ihre Alkohol- und/oder Fettsäurebausteine mindestens teilweise ethylenisch ungesättigt ist. Unter "mindestens teilweise ethylenisch ungesättigt" ist dabei zu verstehen, daß der Anteil der olefinisch ungesättigten Dicarbonsäure- und/oder Alkoholbausteine so hoch ist, daß die Jodzahl des Esters a) mindestens 5 beträgt. Nach oben ist die Jodzahl der Ester a) an ethylenisch ungesättigten Bausteinen in der Praxis durch Werte im Bereich von ca. 300 bis 400 begrenzt. Beispiele für Ester mit einer solch hohen Jodzahl wären etwa solche Dicarbonsäureester, deren Alkoholbausteine Arachidonalkohol sind (Arachidonalkohol = 5,8,11,14-Eicosatetraen-1-ol). Aus Gründen der praktischen Verfügbarkeit setzt man in der Regel solche Ester oder Estergemische a) ein, deren Jodzahl im Bereich von 40 bis 260 liegt.

Die Wahl der Methode zur Bestimmung der Jodzahl ist dabei an sich von untergeordneter Bedeutung. Im Sinne der vorliegenden Erfindung wird jedoch ausdrücklich auf die Methoden nach Hanus bzw. Wijs, die seit langem Bestandteil der Abteilung C-V der "DGF-Einheitsmethoden" sind, sowie die dazu äquivalente neuere Methode nach Fiebig bezug genommen (vergl. Fat Sci. Technol. 1991, Nr.1, S.13-19).

Bei der Herstellung der genannten Umsetzungsprodukte können die Mengenverhältnisse von Ester a) und Sulfierreagens b) in weiten Grenzen variieren. Im Sinne der vorliegenden Erfindung werden die Mengenverhältnisse dieser Komponenten so eingestellt, daß sie im Bereich von 1 : 99 bis 99 : 1 liegen. Dabei ist ein Bereich a) : b) von 10 : 1 bis 1 : 10 und insbesondere ein Bereich von 4: 1 bis 1 : 4 bevorzugt.

Die erfindungsgemäßen sulfierten Substanzen zeichnen sich ganz allgemein durch gute fettende beziehungsweise hydrophobierende Eigenschaften sowie durch eine gute Fogging-Charakteristik aus.

Nach der obigen Definition enthalten die Ester a) einwertige Alkoholbausteine, wobei die Gesamtzahl der C-Atome des Esters, d.h. die Summe der C-Atome von Alkohol- und Fettsäurebausteinen mindestens 16 beträgt.

Beispiele für einwertige Alkohole, die als Alkoholbausteine der Ester a) dienen können und die gesättigt oder ungesättigt sein können, sind Methanol, Ethanol, Propanol, Butanol, Pentanol, Hexanol, Heptanol, Octanol, 2-Ethylhexanol, Pelargonalkohol, Decanol, Undecanol, Laurylalkohol, Tridecanol, Myristinalkohol, Pentadecanol, Palmitylalkohol, Heptadecanol, Stearylalkohol, Nonadecanol, Arachidylalkohol, Heneicosanol, Behenylalkohol, Tricosanol, Lignocerylalkohol, 10-Undecanol, Oleylalkohol, Elaidylalkohol, Ricinolalkohol, Linoleylalkohol, Linolenylalkohol, Gadoleylalkohol, Arachidonalkohol, Erucaalkohol, Brassidylalkohol.

Setzt man solche Ester a) ein, die als Alkoholbausteine sogenannte Guerbet-Alkohole enthalten, so zeichnen sich die daraus hergestellten sulfierten Substanzen dadurch aus, daß sie die Neigung der Leder zur Bildung von Fettausschlag verringern bzw. weitgehend unterdrücken.

Unter Guerbet-Alkoholen sind dabei primäre Alkohole der nachstehenden allgemeinen Formel (I) zu verstehen.

R¹-CH(CH₂OH)-R² (I)

Dabei bedeuten die Reste R¹ und R² unabhängig voneinander eine Alkylgruppe mit 6 bis 12 C-Atomen, mit der Maßgabe, daß die Gesamtzahl der C-Atome pro Guerbetalkoholmolekül im Bereich von 16 bis 24 liegt. Die Guerbet-Alkohole (I) sollen darüber hinaus einen Stockpunkt unterhalb von 6 °C aufweisen. Bevorzugt setzt man solche Guerbet-Alkohole ein, deren Stockpunkt unterhalb von -10 °C liegt. Ganz besonders geeignete Guerbet-Alkohole (I) mit einem Stockpunkt unterhalb von -10 °C sind 2-Hexyldecan-1-ol und 2-Octyldodecan-1-ol.

Die Ester a) können als Vollester oder als Halbester vorliegen. Es ist jedoch auch möglich, Gemische von Vollestern und Halbestern einzusetzen. Eine bevorzugte Ausführungsform der Erfindung besteht darin, solche Estergemische a) einzusetzen, die aus Vollestern und Halbestern der genannten einwertigen Alkohole und Dicarbonsäuren bestehen, wobei das Verhältnis von Vollester und Halbester um Bereich von 1 : 99 bis 99 : 1 und insbesondere 1 : 10 bis 10 : 1 liegt.

In einer weiteren Ausführungsform setzt man die zu sulfierende Komponente a) in Form eines Gemisches aus mindestens einem Dicarbonsäureester a) und mindestens einem Niedrigalkylester einer bei 20 °C flüssigen Fettsäure mit 8 bis 24 C-Atomen ein.

Unter einem Niedrigalkylester ist dabei zu verstehen, daß der Alkoholbaustein des Esters höchstens 8 C-Atome aufweist, wobei der Bereich von 1 bis 4 C-Atomen bevorzugt ist. Der Fettsäureniedrigalkylester kann dabei in bezug auf seine Alkohol- und/oder Fettsäurebausteine gesättigt oder (ethylenisch) ungesättigt sein. Diese Ausführungsform hat den Vorteil, daß dem Fettsäureniedrigalkylester die Aufgabe zukommt, Dicarbonsäureester, die bei 20 °C ganz oder teilweise fest sind, zu verflüssigen oder zumindest in einen homogenen und fließfähigen Zustand zu überführen, so daß sich die Mischung - eventuell nach geringfügiger Erwärmung - leicht rühren läßt.

Eine weitere Ausführungsform der vorliegenden Erfindung besteht darin, daß man solche Ester a) mit dem Sulfierreagens b) umsetzt, die alkoxyliert und/oder epoxidiert sind. Als Bausteine zur Alkoxylierung kommen dabei vor allem Ethylenoxid, Propylenoxid und Butylenoxid oder deren Mischungen in Frage.

Besondere Bedeutung kommt dabei den ethoxylierten Estern zu. Ausdrücklich sei in diesem Zusammenhang darauf hingewiesen, daß im Sinne der vorliegenden Erfindung unter ethoxylierten Estern einerseits solche Esterderivate zu verstehen sind, die sich vom Ester formal durch den Einschub ein oder mehrerer -CH₂-CH₂-O-Gruppen in eine oder beide Esterbindungen unterscheiden - diese Verbindungen lassen sich auf verschiedenen Wegen herstellen, beispielsweise durch Umsetzung der Ester mit Ethylenoxid in Gegenwart eines Katalysators (klassische Ethoxylierung), durch Umsetzung der Ester mit Polyethylenoxid in Gegenwart eines Katalysators oder durch Veresterung einer entsprechenden Dicarbonsäure mit einem Anlagerungsprodukt von Ethylenoxid an den entsprechenden Alkohol -, andererseits solche Esterderivate, die durch Anlagerung von Ethylenoxid an eine primär vorhandene (wie etwa im Fall der Anwesenheit von Äpfelsäurebausteinen) oder einer im Zuge der Sulfierung durch Esterspaltung sekundär gebildeten OH-Gruppe entstehen.

Wie bereits ausgeführt, wird als **Sulfierreagens b)** eine Kombination aus mindestens einer organischen Sulfonsäure b1) und Schwefelsäure b2) eingesetzt; dabei beträgt der Anteil der Schwefelsäure - bezogen auf die Summe von organischer Sulfonsäure und Schwefelsäure - 0,5 bis 80 Gew.-%. Ein Schwefelsäureanteil von 1 bis 40 und insbesondere 5 bis 20 Gew.-% ist jedoch bevorzugt.

Ausdrücklich sei an dieser Stelle klargestellt, daß das Sulfierreagens in Bezug auf die Natur der Schwefelsäure keinen Beschränkungen unterliegt. So kann verdünnte Schwefelsäure, konzentrierte Schwefelsäure oder rauchende Schwefelsäure (konzentrierte Schwefelsäure, die wechselnde Mengen von Schwefeltrioxid gelöst enthält, das sogenannte Oleum) eingesetzt werden. Bevorzugt ist dabei, Schwefelsäure einer Konzentration im Bereich von 96 bis 98 Gew.-% oder Oleum einzusetzen.

Die Auswahl der organischen Sulfonsäuren b1) unterliegt an sich keinen besonderen Einschränkungen. Beispiele für geeignete Sulfonsäuren sind Alkan- oder Halogenalkansulfonsäuren wie Methansulfonsäure, Ethansulfonsäure, Propansulfonsäure, Butansulfonsäure, Chlorsulfonsäure, ferner Naphthalinsulfonsäure, alpha-Sulfo-Fettsäuren (erhältlich z.B. durch Umsetzung von gesättigten Fettsäuren mit gasförmigem Schwefeltrioxid), alpha-Sulfo-Fettsäurealkylester, z.B. -methylester (erhältlich z.B. durch Umsetzung von gesättigten Fettsäurealkylestern mit gasförmigem Schwefeltrioxid), sulfonierte Dimerfettsäuren.

In einer bevorzugten Ausführungsform der vorliegenden Erfindung werden als Komponente b2) jedoch aromatische Sulfonsäuren eingesetzt. Alkylbenzolsulfonsäuren und insbesondere die kommerziell erhältliche lineare Alkylbenzylsulfonsäure sind dabei besonders bevorzugt.

In einer weiteren Ausführungsform werden schließlich die Ester a) mit einem Sulfierreagens b) umgesetzt, das aus einer ternären Kombination von mindestens einer organischen Sulfonsäure b1), Schwefelsäure b2) und mindestens einem Hydrogensulfat b3) (ältere Bezeichnung: Bisulfat) besteht. Geeignete Hydrogensulfate sind Verbindungen der Struktur Alk-HSO₄, wobei "Alk" ein einwertiges Metall oder NH₄ bedeutet. Bevorzugte einwertige Metalle sind die Alkalimetalle, insbesondere Natrium und/oder Kalium.

Ein weiterer Gegenstand der vorliegenden Erfindung ist ein Verfahren zur Herstellung sulfierter Substanzen, wobei man Ester a) von einwertigen Alkoholen mit 1 bis 24 C-Atomen und Dicarbonsäuren mit 2 bis 24 C-Atomen, wobei die Summe der C-Atome der Alkohol- und Fettsäurebausteine der Ester mindestens 16 beträgt, mit einem Sulfierreagens b) umsetzt und das Reaktionsgemisch anschließend neutralisiert, wobei man die Umsetzung bei Temperaturen im Bereich von 20 bis 130 °C durchführt und als Sulfierreagens b) eine Kombination aus mindestens einer organischen Sulfonsäure b1) und Schwefelsäure b2) einsetzt, mit der Maßgabe, daß der Anteil der Schwefelsäure - bezogen auf die Summe von organischer Sulfonsäure und Schwefelsäure - 0,5 bis 80 Gew.-% beträgt.

In Bezug auf einzelne Parameter des Verfahrens (z.B. Einsatzverhältnisse der einzelnen Komponenten) beziehungsweise bevorzugte Bereiche hinsichtlich der Komponenten a) und b) gilt das bereits oben gesagte.

Das erfindungsgemäße Verfahren hat den Vorteil, daß es in der Regel nur mäßige Reaktionstemperaturen erfordert. In den bevorzugten Ausführungsformen liegt die Reaktionstemperatur lediglich im Bereich von 20 bis 80 °C und insbesondere 20 bis 40 °C. In diesen Fällen verläuft die Reaktion nach dem Zusammengeben der Komponenten a) und b) spontan unter Freisetzung von Wärme, so daß in diesen Fällen sogar gekühlt werden muß, um einen kontrollierten Ablauf der Reaktion sicherzustellen.

Bedingt durch die Exothermie der Umsetzung sind die Reaktionszeiten relativ kurz, üblicherweise in der Größenordnung von 1 bis 4 Stunden. Es kann aber - abhängig von der Natur der jeweils eingesetzten Komponente a) - auch gewünscht sein, Reaktionszeiten unterhalb einer Stunde oder oberhalb von 4 Stunden einzustellen.

Das Verfahren wird üblicherweise in der Weise durchgeführt, daß man die Komponenten a) und b) bei etwa 20 °C miteinander mischt. Dabei setzt häufig eine exotherme Reaktion ein - besonders stark in denjenigen Fällen, wo das Sulfierreagens aus einer Mischung von aromatischen Sulfonsäuren und Schwefelsäure besteht.

Die Reaktionsmischung wird nun bei leicht erhöhter Temperatur gerührt. Diese Temperatur richtet sich im wesentlichen nach der Natur der eingesetzten Ester a), wobei einerseits der Gehalt der Ester an primär vorhandenen und/oder im Zuge der Sulfierung durch Esterspaltung gebildeten OH-Gruppen beziehungsweise ihr Gehalt an -C=C-Doppelbindungen, andererseits der Schmelzpunkt der Ester eine Rolle spielt. Sofern möglich, wird das erfindungsgemäße Verfahren bei der geringstmöglichen Temperatur durchgeführt. Diese liegt in Fällen, wo bei 20 °C flüssige Dicarbonsäureester als Komponente a) eingesetzt werden und wo der Anteil der Schwefelsäure im Sulfierreagens b) im bevorzugten Bereich von 1 bis 40 Gew.-% liegt, vorzugsweise im Bereich von 20 bis 40 °C, wobei man häufig extern kühlen muß, um die Temperatur in diesem Bereich zu halten. Bei Estern a), die bei 20 °C oder leicht erhöhter Temperatur nicht hinreichend flüssig sind, oder wo die Reaktion möglichst schnell zum Abschluß gebracht werden soll, kann es jedoch gewünscht sein, die gesamte genannte Temperaturspanne von 20 bis 80 °C beziehungsweise 20 bis 130 °C auszunutzen. In Einzelfällen kann es sogar erwünscht sein, die Reaktion bei höheren Temperaturen (bis ca. 250 °C) durchzuführen, z. B. wenn man als Komponente a) Ester mit hohem Schmelzpunkt einsetzt, jedoch auf den möglichen Zusatz eines Verdünnungsmittels verzichten will oder wenn die Reaktionsgeschwindigkeit noch weiter gesteigert werden soll. Im Bereich dieser hohen Reaktionstemperaturen kann es dabei zweckmäßig sein, die Reaktion unter Luftanschluß in einer Inertgasatmosphäre (z. B. unter Stickstoff) durchzuführen.

Nach der Reaktion der Komponenten a) und b) schließt sich eine Neutralisation an. In der Regel läßt man dazu das Reaktionsgemisch abkühlen und dosiert dann die gewünschte Base zu. Beispiele für geeignete Basen sind Alkalimetallhydroxide, Erdalkalimetallhydroxide, Ammoniumhydroxid, Alkanolamine und Alkylamine beziehungsweise Gemische davon. Bevorzugt sind dabei insbesondere Natriumhydroxid, Kaliumhydroxid und Ammoniumhydroxid. Eine weitere Aufarbeitung der neutralisierten Produkte ist nicht erforderlich.

Die nachfolgenden Beispiele dienen der Erläuterung der Erfindung und sind nicht einschränkend zu verstehen.

### Beispiele

### 1. Substanzen

### 1.1. Dicarbonsäureester a)

E-1: Di-Butyl-Sebacat ("Rilanit DBS", Fa. Henkel/Düsseldorf)
E-2: Di-Talgfettalkohol-Phthalat ("Rilanit DTP", Fa. Henkel/Düsseldorf)
E-3: Di-2-Ethylhexyl-Sebacat ("Rilanit DEHS", Fa. Henkel/Düsseldorf)
E-4: Di-Octyl-Adipat ("Rilanit DOA", Fa. Henkel/Düsseldorf)

### 1.2. Sulfier-Reagentien b)

Alkylbenzolsulfonsäure, ein auch kommerziell erhältliches Produkt, ist zugänglich durch Sulfonierung von linearem Alkylbenzolsulfonat mit gasförmigem Schwefeltrioxid.
- S-1:: Mischung von 99 Gew.-% Alkylbenzolsulfonsäure und 1 Gew.-% konzentrierter (98%-iger) Schwefelsäure
- S-2:: Mischung von 80 Gew.-% Alkylbenzolsulfonsäure und 20 Gew.-% konzentrierter (98%-iger) Schwefelsäure
- S-3:: Mischung von 90 Gew.-% Alkylbenzolsulfonsäure und 10 Gew.-% konzentrierter (98%-iger) Schwefelsäure

### 2. Allgemeine Arbeitsvorschrift zur Sulfierung der Dicarbonsäureester

Etwa gewichtsgleiche Mengen (z.B. je 100 g) Dicarbonsäureester (z.B. E-1 bis E-4) und Sulfierreagens (z.B. S-1 bis S-3) werden bei Raumtemperatur gemischt. In Fällen, wo der eingesetzte Ester bei Raumtemperatur flüssig ist, wird ca. 2 Stunden gerührt, wobei man eine Temperatur im Bereich von 20 bis 40 °C einstellt. Bei exotherm verlaufenden Reaktionen wird entsprechend gekühlt. Bei Estern, die bei 20 °C fest sind (z.B. dem Ester E-2), wählt man eine Reaktionstemperatur, bei der der Ester flüssig ist. Wennmöglich wird dabei eine Temperatur von höchstens 80 °C eingestellt. Anschließend wird die Reaktionsmischung mit einer Base (z.B. 37 gew.-%iger wäßriger Natronlauge) neutralisiert, gegebenenfalls nach vorheriger Kühlung des Gemisches.

## Patentansprüche

1. Verwendung sulfierter Substanzen zur fettenden Ausrüstung von Leder, wobei die sulfierten Substanzen erhältlich sind durch Umsetzung von Estern a) von einwertigen Alkoholen mit 1 bis 24 C-Atomen und Dicarbonsäuren mit 2 bis 24 C-Atomen, wobei die Summe der C-Atome der Alkohol- und Fettsäurebausteine der Ester mindestens 16 beträgt, mit einem Sulfierreagens b) nebst anschließender Neutralisation, **dadurch gekennzeichnet**, daß man als Sulfierreagens b) eine Kombination aus mindestens einer organischen Sulfonsäure b1) und Schwefelsäure b2) einsetzt, mit der Maßgabe, daß der Anteil der Schwefelsäure - bezogen auf die Summe von organischer Sulfonsäure und Schwefelsäure - 0,5 bis 80 Gew.-% beträgt.

2. Verwendung nach Anspruch 1, wobei man die Mengenverhältnisse von Ester a) und Sulfierreagens b) so einstellt, daß sie im Bereich von 1 : 99 bis 99 : 1 liegen.

3. Verwendung nach Anspruch 1 oder 2, wobei die Komponente a) in Bezug auf ihre Alkohol- und/oder Fettsäurebausteine mindestens teilweise ethylenisch ungesättigt ist.

4. Verwendung nach einem der Ansprüche 1 bis 3, wobei es sich bei der organischen Sulfonsäure um eine aromatische Sulfonsäure, vorzugsweise Alkylbenzolsulfonsäure handelt.

5. Verwendung nach Anspruch 4, wobei der Anteil der Schwefelsäure - bezogen auf die Summe von organischer Sulfonsäure und Schwefelsäure - 1 bis 40 Gew.-% beträgt.

6. Verwendung nach einem der Ansprüche 1 bis 5, wobei es sich bei der Alkoholkomponente des Esters a) um einen Guerbet-Alkohol der allgemeinen Formel (I) handelt,
R¹-CH(CH₂OH)-R² (I)
wobei die Reste R¹ und R² unabhängig voneinander eine Alkylgruppe mit 6 bis 12 C-Atomen bedeuten, mit der Maßgabe, daß die Gesamtzahl der C-Atome pro Guerbetalkoholmolekül im Bereich von 16 bis 24 liegt und der Guerbet-Alkohol einen Stockpunkt unterhalb von 6 °C aufweist.

7. Verwendung nach einem der Ansprüche 1 bis 5, wobei man als zu sulfierende Komponente a) ein Gemisch einsetzt bestehend aus
i) mindestens einem Dicarbonsäureester mit 2 bis 24 C-Atomen und
ii) mindestens einem Niedrigalklyester von Fettsäuren mit 8 bis 24 C-Atomen.

8. Verwendung nach einem der Ansprüche 1 bis 7, wobei man solche Ester a) einsetzt, die alkoxyliert und/oder epoxidiert sind.

9. Verfahren zur Herstellung sulfierter Substanzen, wobei man Ester a) von einwertigen Alkoholen mit 1 bis 24 C-Atomen und Dicarbonsäuren mit 2 bis 24 C-Atomen, wobei die Summe der C-Atome der Alkohol- und Fettsäurebausteine der Ester mindestens 16 beträgt, mit einem Sulfierreagens b) umsetzt und das Reaktionsgemisch anschließend neutralisiert, **dadurch gekennzeichnet**, daß man die Umsetzung bei Temperaturen im Bereich von 20 bis 130 °C durchführt und als Sulfierreagens b) eine Kombination aus mindestens einer organischen Sulfonsäure b1) und Schwefelsäure b2) einsetzt, mit der Maßgabe, daß der Anteil der Schwefelsäure - bezogen auf die Summe von organischer Sulfonsäure und Schwefelsäure - 0,5 bis 80 Gew.-% beträgt.

10. Verfahren nach Anspruch 9, wobei man die Mengenverhältnisse von Ester a) und Sulfierreagens b) so einstellt, daß sie im Bereich von 1 : 99 bis 99 : 1 liegen und wobei man als Komponente b) eine aromatische Sulfonsäure, vorzugsweise Alkylbenzolsulfonsäure einsetzt.

## Claims

1. The use of sulfonated substances for the oiling of leather, the sulfonated substances being obtainable by reaction of esters a) of monohydric alcohols containing 1 to 24 carbon atoms and dicarboxylic acids containing 2 to 24 carbon atoms, the sum total of carbon atoms in the alcohol and fatty acid components of the esters being at least 16, with a sulfonating agent b) and subsequent neutralization, characterized in that a combination of at least one organic sulfonic acid b1) and sulfuric acid b2) is used as the sulfonating agent b), with the proviso that the percentage content of the sulfuric acid, based on the sum of organic sulfonic acid and sulfuric acid, is 0.5 to 80% by weight.

2. The use claimed in claim 1, characterized in that the quantity ratios of ester a) to sulfonating agent b) are in the range from 1:99 to 99:1.

3. The use claimed in claim 1 or 2, characterized in that, in regard to its alcohol and/or fatty acid constituents, component a) is at least partly ethylenically unsaturated.

4. The use claimed in any of claims 1 to 3, characterized in that the organic sulfonic acid is an aromatic sulfonic acid, preferably alkyl benzenesulfonic acid.

5. The use claimed in claim 4, characterized in that the percentage content of sulfuric acid, based on the sum of organic sulfonic acid and sulfuric acid, is 1 to 40% by weight.

6. The use claimed in any of claims 1 to 5, characterized in that the alcohol component of the ester a) is a Guerbet alcohol corresponding to general formula (I):
R¹-CH(CH₂OH)-R² (I)
in which R¹ and R² independently of one another represent an alkyl group containing 6 to 12 carbon atoms, with the proviso that the total number of carbon atoms per Guerbet alcohol molecule is 16 to 24 and the Guerbet alcohol has a pour point below 6°C.

7. The use claimed in any of claims 1 to 5, characterized in that component a) to be sulfonated is a mixture of
I) at least one dicarboxylic acid ester containing 2 to 24 carbon atoms and
ii) at least one lower alkyl ester of fatty acids containing 8 to 24 carbon atoms.

8. The use claimed in any of claims 1 to 7, characterized in that alkoxylated and/or epoxidized esters a) are used.

9. A process for the production of sulfonated substances, characterized in that esters a) of monohydric alcohols containing 1 to 24 carbon atoms and dicarboxylic acids containing 2 to 24 carbon atoms, the sum total of carbon atoms in the alcohol and fatty acid components of the esters being at least 16, are reacted with a sulfonating agent b) and the reaction mixture is subsequently neutralized, characterized in that the reaction is carried out at temperatures of 20 to 130°C and in that a combination of at least one organic sulfonic acid b1) and sulfuric acid b2) is used as the sulfonating agent b), with the proviso that the percentage content of the sulfuric acid, based on the sum of organic sulfonic acid and sulfuric acid, is 0.5 to 80% by weight.

10. A process as claimed in claim 8, characterized in that the quantity ratios of ester a) to sulfonating agent b) are in the range from 1:99 to 99:1 and in that an aromatic sulfonic acid, preferably alkyl benzenesulfonic acid, is used as component b).

## Revendications

1. Utilisation de substances sulfatées pour l'apprêtage graissant du cuir, dans laquelle les substances sulfatées peuvent être obtenues par réaction d'esters a) d'alcools monovalents ayant de 1 à 24 atomes de carbone et d'acides dicarboxyliques ayant de 2 à 24 atomes de carbone, pour lesquels la somme des atomes de carbone des éléments constitutifs alcool et acide gras de l'ester s'élève à 16 au moins, comprenant un réactif de sulfatation b) avec une neutralisation consécutive,
caractérisée en ce qu'
on met en oeuvre comme réactif de sulfatation b) une combinaison à base d'au moins un acide sulfonique organique b1) et un acide sulfurique b2) avec la restriction que la quantité d'acide sulfurique - rapporté à la somme d'acide sulfonique organique et d'acide sulfurique - s'élève à 0,5 à 80 % en poids.

2. Utilisation selon la revendication 1,
dans laquelle
les rapports en quantités de l'ester a) et du réactif de sulfatation b) sont ajustés, de sorte qu'ils se situent dans la zone de 1 : 99 à 99 :1.

3. Utilisation selon la revendication 1 ou 2,
dans laquelle
le composant a) est insaturé éthyléniquement au moins partiellement, en référence à leurs éléments constitutifs alcool et/ou acide gras.

4. Utilisation selon l'une des revendications 1 à 3,
dans laquelle
en ce qui concerne l'acide sulfonique organique, il s'agit d'un acide sulfonique aromatique, de préférence d'un acide alkylbenzène sulfonique.

5. Utilisation selon la revendication 4,
dans laquelle
le pourcentage d'acide sulfurique - rapporté à la somme d'acide sulfonique organique et d'acide sulfurique - s'élève à 1 à 40 % en poids.

6. Utilisation selon l'une des revendications 1 à 5,
dans laquelle
en ce qui concerne le composant alcool de l'ester a) il s'agit d'un alcool de Guerbet de formule générale :
R¹-CH(CH₂OH)-R² (I)
dans laquelle les radicaux R¹ et R² indépendamment l'un de l'autre, signifient un groupe alkyle ayant de 6 à 12 atomes de carbone
avec la restriction que le nombre total des atomes de carbone par molécule d'alcool de Guerbet se situe dans la zone de 16 à 24 et que l'alcool de Guerbet possède un point de solidification en dessous de 6°C.

7. Utilisation selon l'une des revendications 1 à 5,
dans laquelle on met en oeuvre comme composant à sulfater a) un mélange consistant en :
i) au moins un ester d'acide dicarboxylique ayant de 2 à 24 atomes de carbone et
ii) au moins un ester d'alkyle inférieur d'acide gras ayant de 8 à 24 atomes de carbone.

8. Utilisation selon l'une des revendications 1 à 7, dans laquelle on met en oeuvre des esters a) qui sont alkoxylés et/ou époxydés.

9. Procédé de préparation de substances sulfatées, dans lequel on fait réagir un ester a) d'alcools monovalents ayant de 1 à 24 atomes de carbone et d'acides dicarboxyliques ayant de 2 à 24 atomes de carbone pour lesquels la somme des atomes de carbone des éléments constitutifs alcool et acide gras de l'ester s'élève à au moins 16, avec un réactif de sulfatation b) et on neutralise ensuite le mélange réactionnel,
caractérisé en ce qu'
on effectue la réaction à des températures dans la plage de 20 à 130°C, et qu'on met en oeuvre comme réactif de sulfatation b) une combinaison d'au moins un acide organique sulfonique b1) et d'acide sulfurique b2) avec la restriction que la proportion d'acide sulfurique - rapporté à la somme d'acide organique sulfonique et d'acide sulfurique - s'élève de 0,5 à 80 % en poids.

10. Procédé selon la revendication 9,
dans lequel
on ajuste les rapports en quantité d'ester a) et de réactif de sulfatation b), de sorte qu'ils se situent dans la zone de 1 : 99 à 99 : 1 et dans lequel on met en oeuvre comme composant b) un acide sulfonique aromatique, de préférence un acide alkylbenzène sulfonique.
